# EUROPÄISCHE PATENTSCHRIFT

(11) **EP 0 891 746 B1**
(45) Veröffentlichungstag und Bekanntmachung des Hinweises auf die Patenterteilung: **21.01.2004**
(21) Anmeldenummer: 98109028.5
(22) Anmeldetag: 18.05.1998
(51) Int. Cl.: A61B 18/08, A61B 18/12

(54) **Hochfrequenzchirurgie-Instrument**
High frequency current surgical instrument
Instrument de chirurgie à courants de haute fréquence

(30) Priorität: 16.07.1997 DE 19730525
(43) Veröffentlichungstag der Anmeldung: 20.01.1999
(73) Patentinhaber: Gebr. Berchtold GmbH & Co., 78532 Tuttlingen (DE)
(72) Erfinder: Fritzsch, Gernod, 78532 Tuttlingen (DE)
(74) Vertreter: Manitz, Gerhart, Dr.

(56) Entgegenhaltungen:
- WO-A-94/08524
- US-A- 3 807 404

## Beschreibung

Die Erfindung betrifft ein Hochfrequenzchirurgie-Instrument nach dem Oberbegriff des Patentanspruchs 1.

Derartige Hochfrequenzchirurgie-Instrumente werden zusammen mit Hochfrequenz-Chirurgiegeräten benutzt, um Biogewebe blutarm zu schneiden, Gewebewucherungen oder krankhafte Körperteile zu entfernen und anderes mehr. Es sind bereits Hochfrequenzchirurgie-Instrumente der eingangs genannten Gattung bekannt, bei welchen monopolare bzw. unipolare Schneidschlingen durch Verschieben des Betätigungsteils relativ zum Handhabungsteil vergrößert oder verkleinert werden können (DE-Gbm 78 15 921; US-PS 5 201 741).

Da bei Hochfrequenz-Chirurgiegeräten häufig mit hoher Hochfrequenzleistung gearbeitet werden muß und die unipolaren Drahtschlingen im allgemeinen sehr dünn sind, ist die Lebensdauer der Schneidschlingen derart begrenzt, daß sie eventuell nur für die Durchführung einer einzigen Operation ausreichen. Bei derartigen Hochfrequenzchirurgie-Instrumenten ist das Auswechseln beschädigter oder zerstörter Schneidschlingen sehr umständlich, weil die Instrumente zum Teil vollständig zerlegt werden müssen, um die das Betätigungsteil bildenden Hubstangen mit den Schneidschlingen auszuwechseln.

Ein weiteres Problem bei bekannten Hochfrequenzchirurgie-Instrumenten mit Schneidschlingen besteht darin, daß eine einmal eingestellte Schneidschlingengröße nur durch besondere Aufmerksamkeit des Operateurs konstant gehalten werden kann. Außerdem muß bei derartigen Instrumenten die Schneidschlinge durch die Hand- oder Fingerkraft des Operateurs am Griff unter einer gewissen Vorspannung gehalten werden, damit sie nicht verrutscht.

Das Ziel der Erfindung besteht darin, ein Hochfrequenzchirurgie-Gerät der eingangs genannten Gattung so auszubilden, daß die Auswechslung abgenutzter oder zerstörter Schneidschlingen schnell und problemlos möglich ist.

Zur Lösung dieser Aufgabe sind die Merkmale des kennzeichnenden Teils des Anspruches 1 vorgesehen. Vorteilhafte Ausführungsformen entnimmt man den Ansprüchen 2 und 3.

Der Erfindungsgedanke ist also darin zu sehen, daß man die Schneidschlinge insbesondere durch Verbindung mit einem Sockel als vom Rest des Instrumentes separierbares Bauteil ausbildet, so daß zwecks Auswechslung einer Schneidschlinge nicht das gesamte Instrument zerlegt werden muß, sondern lediglich die Schneidschlinge durch einfache Handgriffe wie Herausschrauben oder Entriegeln eines Bajonettverschlusses abgenommen und durch eine gleich ausgebildete neue Schneidschlinge ersetzt werden kann.

Eine erste vorteilhafte praktische Ausführungsform von besonders einfacher Gestaltung entnimmt man den Patentansprüchen 4 und 5.

Eine weitere, in axialer Richtung besonders kompakte Ausführungsform ist durch Anspruch 6 gekennzeichnet.

Ein besonders sicheres Ergreifen des Betätigungsteils wird durch die Weiterbildung nach Anspruch 7 gewährleistet. Außerdem wird es bei diesen Ausführungsformen ermöglicht, daß die die Schneidschlinge zusammenziehende Kraft in dem gewünschten Maße durch die Federanordnung aufgebracht wird, so daß der Operateur lediglich steuernd und weniger durch Kraftaufbringung auf das Betätigungsteil einwirken muß. Weiter kann erreicht werden, daß die mehr oder weniger zusammengezogene Schneidschlinge in einer bestimmten erreichten Position festgehalten wird, indem eine nur einseitig sperrende Fixiervorrichtung verwendet wird, weil dann das Zusammenziehen der Schlinge bei nicht wirksamer Fixiervorrichtung erfolgen kann und die Fixierung erst nach Erreichen der gewünschten Endposition der Schneidschlinge erfolgt.

Die auch Anspruch 8 und 9 entnehmbare Federanordnung erfüllt die Doppelfunktion, daß sie die Schneidschlinge zusammenzieht und gleichzeitig die Verriegelung der erreichten Position gewährleistet. Dadurch wird es möglich, die Schneidschlinge unter einer einstellbaren Vorspannung an dem abzutrennenden Organ anzulegen und ein Verrutschen zu verhindern.

Um gezielt an einer bestimmten Stelle schneiden zu können, ist die Ausführungsform nach Anspruch 10 vorgesehen, wo die nicht zum Schneiden bestimmten Teile der Schneidschlinge isoliert ausgebildet sind. Um nach einem Einfahren der Schneidschlinge und anschließendem Wiederausfahren eine vorbestimmte gewünschte Schlingenform zu gewährleisten, ist für die Schneidschlinge zweckmäßig ein Memory-Metalldraht vorgesehen.

Die Erfindung wird im folgenden beispielsweise anhand der Zeichnung beschrieben; in dieser zeigt:
- Figur 1: eine teilweise geschnittene Seitenansicht einer ersten einfachen Ausführungsform eines erfindungsgemäßen Hochfrequenzchirurgie-Instrumentes in der Position mit am weitesten ausgefahrener Schneidschlinge,
- Figur 2: eine entsprechende teilweise geschnittene Seitenansicht bei teilweise eingezogener Schneidschlinge,
- Figur 3: eine Axialschnittansicht eines erfindungsgemäßen Hochfrequenzchirurgie-Instrumentes mit in ihrer Größe veränderbarer einpoliger Schneidschlinge und
- Figur 4: eine entsprechende, jedoch gegenüber Figur 1 vergrößerte Ausschnittsansicht des Instrumentes nach Figur 1 im Bereich der Schneidschlinge und deren Befestigung am Basisteil des Instrumentes, wobei die Schneidschlinge vom Basisteil des Instrumentes gelöst dargestellt ist.

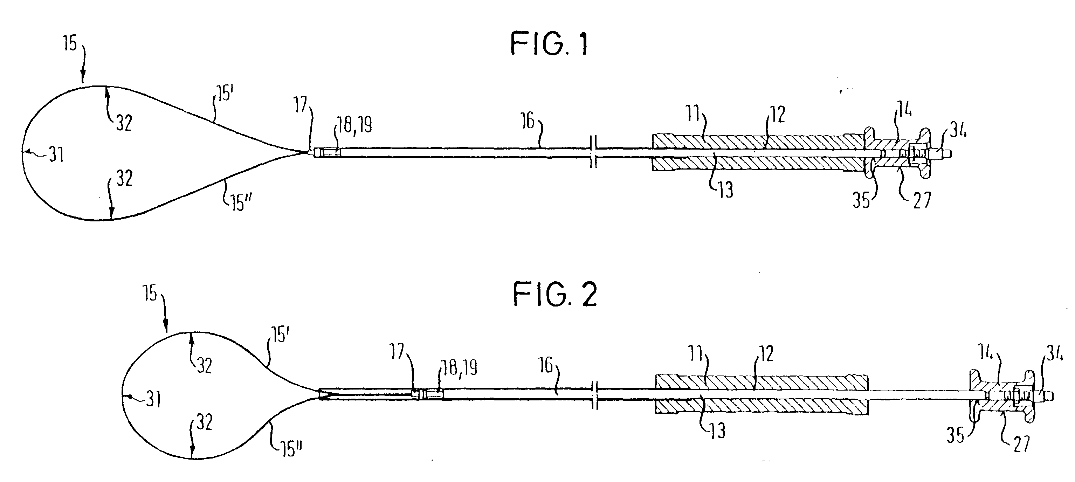

Nach Figur 1 weist ein erfindungsgemäßes Hochfrequenzchirurgie-Instrument ein kreiszylindrisches Handhabungsteil 11 mit einem zentralen von vorn nach hinten durchgehenden Führungskanal 12 auf. In den vorderen Endbereich des Handhabungsteils 11 ist ein mit dem Führungskanal 12 koaxiales Rohr 16 so eingesetzt, daß der Führungskanal 12 und der Innenraum des Rohres 16 bündig ineinander übergehen und zumindest im wesentlichen den gleichen Querschnitt aufweisen.

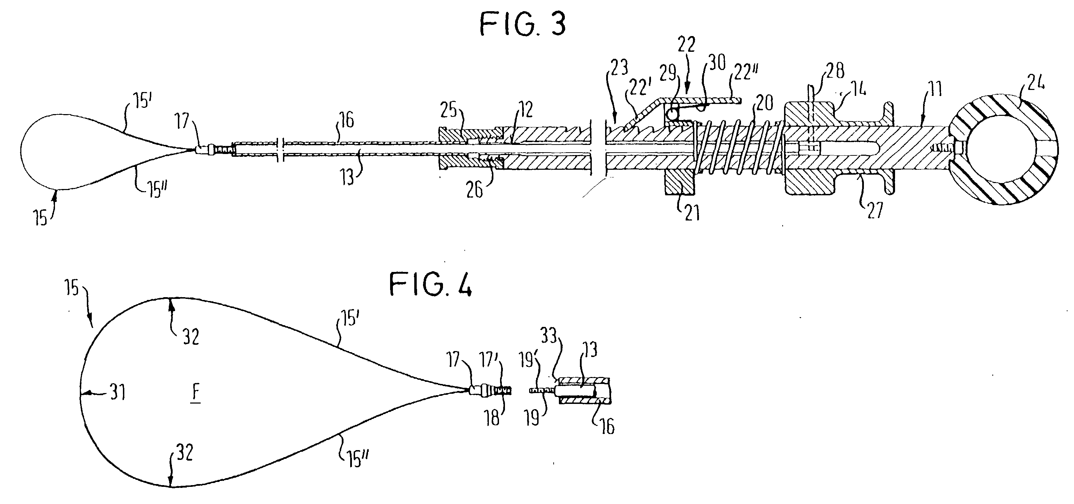

In dem Führungskanal 12 und dem Rohr 16 befindet sich ein Schaft 13, der im Führungskanal 12 und im Rohr 16 axial verschiebbar ist. Am vorderen Ende des Schaftes 13 ist über eine später anhand von Figur 4 im einzelnen beschriebene Gewindeverbindung 18, 19 ein Sockel 17 lösbar befestigt, der ebenso wie die Gewindeverbindung 18, 19 so dimensioniert ist, daß er innerhalb des Rohres 16 und gegebenenfalls des Führungskanals 12 Platz hat und axial verschiebbar ist.

In dem Sockel 17 sind die beiden Schenkel 15', 15" einer Schneidschlinge 15 befestigt, die aus dem vorderen Ende des Rohres 16 mehr oder weniger vorsteht, je nachdem, wie weit der Sockel 17 in das Rohr 16 hineingezogen ist (Figuren 1, 2).

Die Schneidschlinge 15 besteht aus einem elastischen Draht, der in einem mittleren Schneidbereich 31 freiliegt und im übrigen mit einer Isolierung 32 versehen ist.

Am hinteren Ende steht der Schaft 13 aus dem hinteren Ende des Führungskanals 12 vor und tritt dort in eine zentrale Bohrung 35 eines Betätigungsteils 14 ein, welches beim Ausführungsbeispiel nach den Figuren 1 und 2 im wesentlichen die Form eines Kreiszylinders mit Flanschen an beiden Enden besitzt, wodurch eine rundumlaufende Griffmulde 27 gebildet wird.

Innerhalb des Betätigungsteils 14 ist der Schaft 13 elektrisch leitend mit einem vorzugsweise als Steckkontakt ausgebildeten Hochfrequenzkontakt 34 verbunden, welcher nach hinten vorsteht und dort über ein geeignetes, aufgestecktes Gegenstück mit einem Hochfrequenzgenerator verbindbar ist.

Die Funktion des beschriebenen Instrumentes ist wie folgt:

Sofern nach Figur 1 das Betätigungsteil 14 so weit nach vorn verschoben ist, daß es am hinteren Ende des Handhabungsteils 11 anliegt, steht der Sockel 17 geringfügig über das vordere Ende des Rohres 16 vor, und die Schneidschlinge 15 nimmt ihre größte Gestalt an.

Sobald während einer elektrochirurgischen Operation die Schneidschlinge 15 einen bestimmten Gewebebereich umfaßt hat, zieht der Operateur das Betätigungsteil 14 aus der Position nach Figur 1 relativ zum Handhabungsteil 11 zurück, und zwar beispielsweise in die Position nach Figur 2, wo die beiden Schenkel 15', 15" teilweise in das Rohr 16 hineingezogen sind und die Rest-Schneidschlinge dadurch einen wesentlich kleineren Querschnitt umfaßt. Auf diese Weise kann von der Schneidschlinge 15 erfaßtes Gewebe geklemmt und anschließend durch Anlegen einer Hochfrequenzspannung an die Schneidschlinge 15 optimal geschnitten werden.

Nach einer Operation kann das Betätigungsteil 14 wieder nach vorne geschoben werden, wodurch die Schneidschlinge 15 relativ einfach wieder vom Gewebe freikommen kann.

Nach Figur 3 weist eine weitere Ausführungsform des erfindungsgemäßen Hochfrequenzchirurgie-Instrumentes ein im wesentlichen kreiszylindrisch ausgebildetes Handhabungsteil 11 auf, welches am hinteren Ende durch einen Daumeneingriffsring 24 abgeschlossen ist und einen nach vorn offenen, im wesentlichen kreiszylindrischen, zentralen Führungskanal 12 aufweist. An das vom Daumeneingriffsring 24 abgewandte, vordere Ende des Führungskanals 12 schließt sich eine Überwurfmutter 25 an, die auf einen vorderen Gewindestutzen 26 des Handhabungsteils 11 aufgeschraubt ist und ein vorn vorstehendes Rohr 16 trägt. Das Handhabungsteil 11 und das Rohr 16 sollen aus Isoliermaterial bestehen.

Auf dem hinteren kreiszylindrischen Teil des Handhabungsteils 11 befindet sich ein ringartiges Betätigungsteil 14, welches auf dem Handhabungsteil 11 axial verschiebbar angeordnet ist und eine Griffmulde 27 für die Finger einer Bedienungsperson aufweist.

Innerhalb des Führungskanals 12 bzw. der Überwurfmutter 25 des Rohres 16 befindet sich ein metallischer Schaft 13, welcher am rückwärtigen Ende durch nicht dargestellte Schlitze im Handhabungsteil 11 mit dem Betätigungsteil 14 bevorzugt formschlüssig verbunden ist.

Durch das vorzugsweise isoliert ausgebildete Betätigungsteil 14 erstreckt sich radial außerdem eine nur angedeutete Hochfrequenzleitung 28, die an einen nicht dargestellten Hochfrequenzgenerator angeschlossen ist und von diesem eine Hochfrequenzverbindung zum hinteren Ende des MetallSchaftes 13 herstellt.

Im vorderen Bereich des Handhabungsteils 11 ist auf der Außenseite eine sich axial erstreckende Rastverzahnung 23 vorgesehen, die mit einer Sperrklinke 22 zusammenwirkt, welche an einem axial verschiebbar auf dem Handhabungsteil 11 sitzenden Fixierring 21 um eine Querachse 29 verschwenkbar und durch eine Rastfeder 30 auf die Rastverzahnung 23 zu vorspannbar angeordnet ist. Der Rasthebel 22 weist einen vorderen Rastarm 22' und einen hinteren Betätigungsarm 22" auf.

Das vordere Ende des Schaftes 13 ist nach Figur 4 mit einem Zapfen 19' verringerten Durchmessers versehen, der ein Außengewinde 19 aufweist.

Auf den Schraubzapfen 19' ist nach den Figuren 3 und 4 eine mit Innengewinde 18 versehene Schraubhülse 17' aufgeschraubt bzw. aufschraubbar, welche den hinteren Teil eines im wesentlichen kreiszylindrischen Sockels 17 bildet, an dem die beiden Schenkel 15', 15" einer elastischen Schneidschlinge 15 zusammengeführt und in der aus der Zeichnung ersichtlichen Weise fixiert sind.

Die Schneidschlinge 15 besteht aus einem elastischen Draht, der in der Mitte bei 31 nach außen freiliegt und bei 32 mit einer Isolierung versehen ist.

Zwischen dem Fixierring 21 und dem Betätigungsring 14 befindet sich auf dem Betätigungsteil 11 eine Schraubenfeder 20.

Die Funktion der zweiten Ausführungsform des Hochfrequenzchirurgie-Instrumentes nach den Figuren 3 und 4 ist wie folgt:

Zum Montieren einer mit einem Sockel 17 versehenen Schneidschlinge 15 wird der Schaft 13 unter Lösung des Rasthebels 22 aus der Rastverzahnung 23 so weit nach vorne geschoben, bis der Schraubzapfen 19' auf dem Rohr 16 nach vorn vorsteht. Nunmehr kann der Sockel 17 mit der Schneidschlinge 15 problemlos auf den Schraubzapfen 19' aufgeschraubt werden.

Anschließend kann die Schneidschlinge 15 mit dem Sockel 17 durch Zurückziehen des Betätigungsringes 14 auf dem Handhabungsteil 11 nach hinten mehr oder weniger teilweise in das Rohr 16 hineingezogen werden. Der Außendurchmesser des Sockels 17 ist an keiner Stelle größer, als daß dieses Zurückziehen in den Innenraum des Rohrs 16 hinein möglich bleibt.

Sobald der Sockel 17 sich innerhalb des Rohres 16 befindet, kommen die Schenkel 15', 15" der Schneidschlinge 15 mit dem Vorderrand 33 (Figur 2) des Rohres 16 in Kontakt, was dazu führt, daß die Fläche F des Innenraumes der Schneidschlinge 15 zunehmend verkleinert wird, je weiter der Sockel 17 in das Rohr 16 hineingezogen wird. Diese Bewegung wird von der Bedienungsperson dadurch ausgeführt, daß ihr Daumen sich innerhalb des Daumeneingriffringes 24 und Zeige- und Mittelfinger sich in der Griffmulde 27 des Betätigungsringes 14 befinden.

Bei dieser Bewegung wird über die leicht auf Zug beanspruchte Schraubenfeder 20 der Fixierring 21 mitgenommen, und der Rasthebel 22 bzw. der Rastarm 22' springt dabei selbsttätig von einem Zahn der Rastverzahnung 23 in den nächsten Zahn, bis die gewünschte Verkleinerung der Fläche F der Schneidschlinge 15 erreicht ist.

Läßt der Operateur nunmehr den Betätigungsring 14 los, so versucht sich die elastische Schneidschlinge 15 etwas zu erweitern und zieht dabei den Betätigungsring 14 geringfügig nach vorn. Hierbei wird der Fixierring 21 mitgenommen, bis der Rastarm 22' in die Zahnlücke, in deren Bereich er sich befindet, eingerastet ist. Sobald sich nun die elastische Ausziehkraft der Schneidschlinge 15 und die Rückstellkraft der Schraubenfeder 20 die Waage halten, bleibt die erhaltene Größe der Innenfläche der Schneidschlinge 15 aufrecht erhalten, so daß der Operateur das Instrument zeitweise loslassen und sich anderen Tätigkeiten widmen kann. Von nun an ist eine weitere Bewegung der Stange 13 nach vorn nur noch durch Überwindung der Kraft der Feder 20 möglich.

Soll nun die Schneidschlinge wieder vergrößert werden, so braucht der Operateur lediglich den Betätigungsarm 22" des Rasthebels 22 nach unten zu drücken, worauf der Rastarm 22' aus der Rastverzahnung 23 ausklinkt und nunmehr die Gesamtanordnung aus Betätigungsring 14, Schraubenfeder 20 und Fixierring 21 nach vorn verschoben werden kann, wobei die Stange 13 die Schneidschlinge 15 mehr und mehr aus dem Rohr 16 nach vorne herausbewegt, bis schließlich die am weitesten vorstehende Position nach Figur 3 erreicht ist.

### Bezugszeichenliste

- 11: Handhabungsteil
- 12: Führungskanal
- 13: Schaft
- 14: Betätigungsring
- 15: Schneidschlinge
- 15': Schenkel
- 15": Schenkel
- 16: Rohr
- 17: Sockel
- 17': Hülse
- 18: Innengewinde
- 19: Außengewinde
- 19': Schraubzapfen
- 20: Feder
- 21: Fixierring
- 22: Rasthebel
- 22': Rastarm
- 22": Betätigungsarm
- 23: Rastverzahnung
- 24: Daumeneingriffsring
- 25: Überwurfmutter
- 26: Gewindestutzen
- 27: Griffmulde
- 28: Hochfrequenzleitung
- 29: Querachse
- 30: Rastfeder
- 31: blanke Stelle
- 32: isolierte Stellen
- 33: Vorderrand
- 34: Hochfrequenzkontakt
- 35: Bohrung

## Patentansprüche

1. Hochfrequenzchirurgie-Instrument mit einem Handhabungsteil (11), welches einen zumindest einseitig offenen Führungskanal (12) und ein darin axial verschiebbar geführtes, von einer Hochfrequenzspannung beaufschlagtes Halteelement (13) aufweist, welches durch ein relativ zum Handhabungsteil (11) axial verstellbares Betätigungsteil (14) axial verschiebbar ist und an seinem freien Ende mit einer vorzugsweise elastischen Schneidschlinge (15) elektrisch leitend verbunden ist, derart, daß durch axiale Verstellung des Betätigungsteils (14) die Schneidschlinge (15) mehr oder weniger weit in den Führungskanal (12) oder ein ihn nach außen fortsetzendes Rohr (16) hineinziehbar bzw. aus dem Führungskanal (12) oder das ihn nach außen fortsetzende Rohr (16) herausschiebbar und dadurch die Schlingengröße veränderbar ist,
**dadurch gekennzeichnet,**
**daß** die beiden Schenkel (15', 15") der Schneidschlinge (15) an einem im Führungskanal (12) bzw. Rohr (16) geführten, das Halteelement bildenden Schaft (13) lösbar befestigt sind, so daß eine schnelle und problemlose Auswechslung der Schneidschlinge möglich ist.

2. Instrument nach Anspruch 1,
**dadurch gekennzeichnet,**
**daß** die Schenkel (15, 15') zu einem gemeinsamen Sockel (17) geführt sind, der seinerseits mit dem Schaft (13) lösbar verbindbar und so dimensioniert ist, daß er mit diesem innerhalb des Führungskanals (12) bzw. Rohres (16) verschiebbar ist, wobei insbesondere der Sockel (17) über eine Gewindeverbindung (18, 19) oder über eine Bajonettverschlußverbindung mit dem Schaft (13) lösbar verbindbar ist.

3. Instrument nach Anspruch 1 oder 2,
**dadurch gekennzeichnet,**
**daß** der Schaft (13) in dem Führungskanal (12) bzw. Rohr (16) so weit nach vorn verschiebbar ist, daß sein vorderes Verbindungsende (19) sich zumindest so nahe am vorderen Ende des Führungskanals (12) bzw. Rohres (16) befindet und vorzugsweise aus diesem Ende vorsteht, daß die Schenkel (15', 15") der Schneidschlinge (15) bzw. der Sockel (17) problemlos von dem Verbindungsende (19) gelöst bzw. mit ihm verbunden werden können.

4. Instrument nach einem der vorhergehenden Ansprüche,
**dadurch gekennzeichnet,**
**daß** das Betätigungsteil (14) hinter dem Handhabungsteil (11) angeordnet ist, wobei bevorzugt der Führungskanal (12) bis zum hinteren Ende des Handhabungsteils (11) durchgeht und sich der Schaft (13) durch das hintere Ende des Führungskanals (12) zum Betätigungsteil (14) erstreckt und mit diesem fest verbunden ist,
und/ oder der Schaft (13) sich in das Betätigungsteil (14) hineinerstreckt und von dort mit einem Hochfrequenzkontakt, insbesondere Hochfrequenzsteckkontakt (34) elektrisch leitend verbunden ist, der von außen mit einem vom Hochfrequenzgenerator kommenden Gegenstück verbindbar ist.

5. Instrument nach Anspruch 4,
**dadurch gekennzeichnet,**
**daß** das Betätigungsteil (14) bei am weitesten geöffneter Schneidschlinge (15) am hinteren Ende des Handhabungsteils (11) anliegt.

6. Instrument nach einem der Ansprüche 1 bis 3,
**dadurch gekennzeichnet,**
**daß** das Betätigungsteil als Betätigungsring (14) ausgebildet ist, der axial gleitbar auf dem Handhabungsteil (11) angeordnet und durch einen Längsschlitz hindurch mit dem innerhalb des Handhabungsteils (11) axial geführten Schaft (13) verbunden ist.

7. Instrument nach einem der vorhergehenden Ansprüche,
**dadurch gekennzeichnet,**
**daß** das Betätigungsteil (14) eine vorzugsweise rundumlaufende Griffmulde (27) aufweist und/oder das Betätigungsteil (14) in Richtung einer Schlingenverkleinerung durch eine Federanordnung (20) vorgespannt ist und/oder ein Fixiermittel (21, 22, 23) für die in eine bestimmte Position eingezogene Schneidschlinge (15) vorgesehen ist, wobei insbesondere das Fixiermittel aus einer einseitig sperrenden Fixiervorrichtung (21, 22, 23) besteht, die sich beim Verkleinern der Schneidschlinge (15) entsprechend mitverschiebt, jedoch nur durch eine handbetätigte Entsperrung (22) im die Schneidschlinge (15) vergrößernden Sinne verschiebbar ist, und daß vorzugsweise die Federanordnung zwischen dem Fixiermittel (21, 22, 23) und dem Betätigungsteil (14) angeordnet ist, wobei insbesondere das Fixiermittel aus einem auf dem Handhabungsteil (11) verschiebbar geführten, von einer Feder in Sperrichtung beaufschlagbaren Fixierring (21), einer daran angeordneten Rastklinke (22) und einer axialen Rastzahnung (23) auf dem Handhabungsteil (11) besteht und dabei zweckmäßigerweise zwischen dem Fixierring (21) und dem Betätigungsteil (10) eine Schraubenfeder (20) angeordnet ist.

8. Instrument nach einem der vorhergehenden Ansprüche,
**dadurch gekennzeichnet,**
**daß** das Handhabungsteil (11) zumindest im wesentlichen kreiszylindrisch ausgebildet ist und/oder daß die Schraubenfeder (20) um das Handhabungsteil (11) herum angeordnet ist.

9. Instrument nach Anspruch 7 oder 8,
**dadurch gekennzeichnet,**
**daß** die Federanordnung (20) sich beim Einziehen der Schneidschlinge (15) in im wesentlichen entspannten Zustand befindet bzw. gerade so weit in Zugrichtung gespannt ist, daß sie das Fixiermittel (21, 22, 23) mitnehmen kann und anschließend durch die elastische Kraft der Schneidschlinge (15) derart in Druckrichtung gespannt wird, daß die Fixiermittel (21) wirksam werden.

10. Instrument nach einem der vorhergehenden Ansprüche,
**dadurch gekennzeichnet,**
**daß** die Schneidschlinge (15) in ihrem wirksamen Bereich (31), der vorzugsweise vorne liegt, eine metallische Oberfläche aufweist und im übrigen (32) isoliert ist und/oder daß die Schneidschlinge zumindest teilweise und vorzugsweise insgesamt aus einem Memory-Metalldraht besteht.

## Claims

1. High frequency surgical instrument comprising a handling part (11) which has a guide channel (12) which is open at at least one end and a holding element (13) which is axially displaceably guided therein and to which a high frequency voltage is applied, with the holding element (13) being axially displaceable by means of an actuating part (14) axially displaceable relative to the handling part (11) and being electrically conductively connected at its free end to a preferably elastic cutting loop (15) in such a way that, through axial adjustment of the actuating part (14), the cutting loop (15) can be drawn to a greater or lesser degree into the guide channel (12), or into a tube (16) which continues it outwardly, or can be pushed out of the guide channel (12), or out of the tube (16) continuing it outwardly, and with the loop size being able to be changed thereby,
**characterized in that** the two limbs (15', 15") of the cutting loop (15) are releasably secured to a shaft (13) which is guided in the guide channel (12) and/or in the tube (16) and forms the holding element, such that a fast and problem-free replacement of the cutting loop is possible.

2. Instrument in accordance with claim 1, **characterized in that** the limbs (15, 15') are led to a common base (17) which is in turn releasably connectable to the shaft (13) and is dimensioned such that it is displaceable with the shaft within the guide channel (12) and/ or within the tube (16), with the base (17) in particular being releasably connectable to the shaft (13) via a threaded connection (18, 19) or via a bayonet connection.

3. Instrument in accordance with claim 1 or claim 2, **characterized in that** the shaft (13) can be displaced forwardly in the guide channel (12) and / or in the tube (16) to such an extent that its front connecting end (19) is located at least so close to the front end of the guide channel (12) or tube (16) respectively, and preferably projects out of this end, such that the limbs (15', 15") of the cutting loop (15) or the base (17) can be released from the connection end (19) or connected to it without problem.

4. Instrument in accordance with any one of the preceding claims, **characterized in that** the actuating part (14) is arranged behind the handling part (11), with the guide channel (12) preferably passing up to the rear end of the handling part (11) and the shaft (13) extending through the rear end of the guide channel (12) to the actuating part (14) and being fixedly connected to the latter; and/or **in that** the shaft (13) extends into the actuating part (14) and is electrically conductively connected from there to a high frequency contact, in particular to a high frequency plug contact (34) which can be connected from the outside to a counterpiece coming from the high frequency generator.

5. Instrument in accordance with claim 4, **characterized in that** the actuating part (14) contacts the rear end of the handling part (11) when the cutting loop (15) is open to the greatest degree.

6. Instrument in accordance with any one of claims 1 to 3, **characterized in that** the actuating part is formed as an actuating ring (4) which is axially slidably arranged on the handling part (11) and is connected through an elongate slot to the shaft (13) which is axially guided inside of the handling part (11).

7. Instrument in accordance with any one of the preceding claims, **characterized in that** the actuating part (14) has a gripping depression (27) which preferably extends around it, and/or the actuating part (14) is biased by a spring arrangement (20) in the direction of a reduction of the loop, and/ or a fixing means (21, 22, 23) is provided for the cutting loop (15) when drawn into a specific position, with the fixing means in particular comprising a fixing device (21, 22, 23) which blocks at one side and which is correspondingly co-displaced on reduction of the size of the cutting loop (15) but is only displaceable in the sense of an increase in the size of a cutting loop (15) by a hand actuated deblocking device (22); and **in that** the spring arrangement is preferably arranged between the fixing means (21, 22, 23) and the actuating part (14), with the fixing means in particular comprising a fixing ring (21) which is displaceably guided on the handling part (11) and which is loaded by a spring in the blocking direction, a latch pawl (22) arranged on the fixing ring and an axial latch tooth arrangement (23) on the handling part (11) and with a coil spring (20) expediently being arranged between the fixing ring (21) and the actuating part (10).

8. Instrument in accordance with any one of the preceding claims, **characterized in that** the handling part (11) is at least substantially of right cylindrical shape, and/or **in that** the coil spring (20) is arranged around the handling part (11).

9. Instrument in accordance with claim 7 or claim 8, **characterized in that** the spring arrangement (20) is in the essentially relaxed state on pulling in the cutting loop (15), or has been tensioned in the pulling direction just sufficiently that it can move the fixing means (21, 22, 23) with it, and is subsequently tensioned in the compression direction by the elastic force of the cutting loop (15) such that the fixing means (21) become active.

10. Instrument in accordance with any one of the preceding claims, **characterized in that** the cutting loop (15) has a metallic surface in its effective zone (31), which is preferably forwardly disposed, and is insulated elsewhere (32); and/or **in that** the cutting loop consists at least in part and preferably fully of a memory metal wire.

## Revendications

1. Instrument chirurgical à haute fréquence avec un élément de maniement (11) qui comporte un canal de guidage (12) ouvert au moins d'un côté et un élément de retenue (13) qui est guidé de manière à être déplacé axialement à l'intérieur, qui est alimenté avec une tension à haute fréquence, qui peut être déplacé axialement par un élément d'actionnement (14) mobile axialement par rapport à l'élément de maniement (11) et qui est relié électriquement par son extrémité libre à une boucle coupante (15) de préférence élastique, de telle sorte que, par un ajustement axial de l'élément d'actionnement (14), la boucle coupante (15) peut être tirée plus ou moins loin dans le canal de guidage (12) ou dans un tube (16) continuant celui-ci à l'extérieur ou être poussée hors du canal de guidage (12) ou du tube (16) continuant celui-ci à l'extérieur et la dimension de la boucle peut ainsi être modifiée,
**caractérisé en ce que**
les deux côtés (15', 15") de la boucle coupante (15) sont fixés amovibles à une tige (13) guidée dans le canal de guidage (12) ou dans le tube (16) et formant l'élément de retenue de manière à permettre un remplacement rapide et sans problème de la boucle coupante.

2. Instrument selon la revendication 1,
**caractérisé en ce que**
les côtés (15, 15') sont guidés vers un socle commun (17) qui quant à lui peut être assemblé amovible à la tige (13) et est dimensionné de telle sorte qu'il peut être déplacé avec celle-ci à l'intérieur du canal de guidage (12) ou du tube (16), le socle (17) pouvant être assemblé amovible à la tige (13) notamment par l'intermédiaire d'un assemblage à vis (18, 19) ou d'un assemblage à baïonnette.

3. Instrument selon la revendication 1 ou 2,
**caractérisé en ce que**
la tige (13) est déplaçable dans le canal de guidage (12) ou dans le tube (16) si loin vers l'avant que son extrémité d'assemblage avant (19) se trouve au moins si près de l'extrémité avant du canal de guidage (12) ou du tube (16) et de préférence dépasse de cette extrémité, que les côtés (15', 15") de la boucle coupante (15) ou du socle (17) peuvent être détachés sans problème de l'extrémité d'assemblage (19) ou peuvent être assemblés sans problème à celle-ci.

4. Instrument selon l'une des revendications précédentes,
**caractérisé en ce que**
l'élément d'actionnement (14) est placé derrière l'élément de maniement (11), le canal de guidage (12) allant de préférence jusqu'à l'extrémité arrière de l'élément de maniement (11) et la tige (13) s'étendant à travers l'extrémité arrière du canal de guidage (12) vers l'élément d'actionnement (14) et étant assemblée solidaire de celui-ci, et/ou **en ce que** la tige (13) s'étend dans l'élément d'actionnement (14) et est reliée électriquement à partir de là à un contact à haute fréquence, notamment à une fiche à haute fréquence (34), qui peut être relié de l'extérieur à une pièce complémentaire provenant du générateur de haute fréquence.

5. Instrument selon la revendication 4,
**caractérisé en ce que**
l'élément d'actionnement (14) se trouve contre l'extrémité arrière de l'élément de maniement (11) lorsque la boucle coupante (15) est la plus largement ouverte.

6. Instrument selon l'une des revendications 1 à 3,
**caractérisé en ce que**
l'élément d'actionnement est conçu comme un anneau d'actionnement (14) qui est placé de manière à pouvoir glisser axialement sur l'élément de maniement (11) et qui est assemblé à travers une fente longitudinale à la tige (13) guidée axialement à l'intérieur de l'élément de maniement (11).

7. Instrument selon l'une des revendications précédentes,
**caractérisé en ce que**
l'élément d'actionnement (14) comporte une poignée concave (27) qui en fait le tour de préférence et/ou l'élément d'actionnement (14) est précontraint en vue d'une réduction de boucle par un dispositif à ressort (20) et/ou un moyen de fixation (21, 22, 23) est prévu pour la boucle coupante (15) tirée dans une certaine position, le moyen de fixation étant constitué notamment d'un dispositif de fixation (21, 22, 23) qui bloque d'un côté et qui se déplace en même temps en conséquence lors de la réduction de la boucle coupante (15) mais qui ne peut être déplacé que par un déblocage manuel (22) dans le sens d'un agrandissement de la boucle coupante (15) et **en ce que** le dispositif à ressort est placé de préférence entre le moyen de fixation (21, 22, 23) et l'élément d'actionnement (14), le moyen de fixation étant constitué notamment d'un anneau de fixation (21), qui est guidé de manière à pouvoir être déplacé sur l'élément de maniement (11) et qui peut être soumis à un ressort dans la direction de blocage, d'un cliquet (22) agencé dessus et d'une denture axiale (23) sur l'élément de maniement (11) et un ressort hélicoïdal (20) étant agencé alors de façon appropriée entre l'anneau de fixation (21) et l'élément d'actionnement (10).

8. Instrument selon l'une des revendications précédentes,
**caractérisé en ce que**
l'élément de maniement (11) est conçu au moins globalement cylindrique circulaire et/ou **en ce que** le ressort hélicoïdal (20) est placé autour de l'élément de maniement (11).

9. Instrument selon la revendication 7 ou 8,
**caractérisé en ce que**
le dispositif à ressort (20) lors du retrait de la boucle coupante (15) se trouve dans un état globalement détendu ou est juste tendu si loin dans la direction de traction qu'il peut entraîner le moyen de fixation (21, 22, 23) et qu'il est ensuite tendu par la force élastique de la boucle coupante (15) dans la direction de poussée de telle sorte que les moyens de fixation (21) deviennent actifs.

10. Instrument selon l'une des revendications précédentes,
**caractérisé en ce que**
la boucle coupante (15) comporte une surface métallique dans sa zone active (31) qui se trouve de préférence en avant et qu'elle est isolée pour le reste (32) et/ou **en ce que** la boucle coupante est constituée au moins partiellement et de préférence entièrement d'un fil métallique à mémoire de forme.
